# FASCICULE DE BREVET EUROPEEN

(11) **EP 3 185 694 B1**
(45) Date de publication et mention de la délivrance du brevet: **04.09.2019**
(21) Numéro de dépôt: 15763058.3
(22) Date de dépôt: 26.08.2015
(51) Int. Cl.: A23J 1/00, C02F 3/32, C12N 1/12, C12P 5/00

(54) **NOUVEAU PROCEDE DE CULTURE D'ALGUES, PARTICULIEREMENT DE MICROALGUES**
NEUARTIGES VERFAHREN ZUR KULTIVIERUNG VON ALGEN, INSBESONDERE MIKROALGEN
NOVEL METHOD FOR CULTURE OF ALGAE, IN PARTICULAR MICROALGAE

(30) Priorité: 26.08.2014 FR 1457999
(43) Date de publication de la demande: 05.07.2017
(73) Titulaire: Fermentalg, 33500 Libourne (FR)
(72) Inventeur: CALLEJA, Pierre, 33500 Libourne (FR); LETERRIER, Marina, F-33500 Libourne (FR)
(74) Mandataire: Be IP
(86) Numéro de dépôt international: PCT/FR2015/052273
(87) Numéro de publication internationale: WO 2016/030630

(56) Documents cités:
- WO-A1-2011/035166
- WO-A1-2013/136027
- WO-A2-2006/085144
- WO-A2-2013/063075
- WO-A2-2014/074770
- WO-A2-2015/004403
- CN-A- 103 352 006
- KR-A- 20110 072 830
- KR-A- 20140 044 419
- ELEONORA SFORZA ET AL: "Adjusted Light and Dark Cycles Can Optimize Photosynthetic Efficiency in Algae Growing in Photobioreactors", PLOS ONE, vol. 7, no. 6, 20 juin 2012 (2012-06-20), page e38975, XP055103017, ISSN: 1932-6203, DOI: 10.1371/journal.pone.0038975

## Description

La présente demande concerne le domaine de la culture des algues, particulièrement des microalgues.

L'invention s'adresse particulièrement à un procédé de culture d'algues, avantageusement de microalgues, caractérisé en ce que les conditions particulières de la culture en termes d'éclairage et de nutriments permettent d'obtenir une biomasse constituée de microalgues présentant au moins une quantité en chlorophylle faible, avantageusement très faible et une quantité en agents antioxydants élevée.

Certaines microalgues sont des êtres mixotrophes, capable à la fois d'hétérotrophie (absorption de matière organique dans le milieu de culture) et d'autotrophie (utilisation de la lumière pour capter le carbone via la photosynthèse). La mixotrophie est également appelée photo-hétérotrophie. La notion de mixotrophie est étendue à l'utilisation de la lumière non seulement pour la photosynthèse mais également comme signal lumineux pouvant induire une réponse du métabolisme : par exemple la synthèse de pigments.

La mixotrophie à dominante hétérotrophe permet de produire des molécules d'origine algale en couplant à la fois les avantages de l'autotrophie et de l'hétérotrophie. Elle consiste à introduire une composante lumineuse de faible intensité et de courte durée. Comme en hétérotrophie, le substrat organique nourrit les microalgues pour produire de grandes quantités de biomasse mais cette fois le chloroplaste et autres organites capteurs d'énergie lumineuse de la cellule sont activés.

Ces capteurs d'énergie lumineuse ou photo récepteurs sont présents dans des organites comme le stigma qui est considéré comme un oeil primitif qui permet aux cellules flagellées qui en disposent de se déplacer en fonction de la quantité et de la qualité de la lumière. Les autres organites photosensibles sont le chloroplaste, le chronoplaste ou le chromoplaste.

Ces photorécepteurs permettent d'augmenter la productivité de la cellule ainsi que de permettre la synthèse de toutes les molécules pouvant être métabolisées par une microalgue. Les molécules d'intérêt, produites par les microalgues, présentent un intérêt industriel majeur particulièrement dans les domaines de la nutrition, de la cosmétique, de la chimie verte et de l'énergie.

Ces molécules sont variées telles que par exemple des carbohydrates, des protéines, des acides aminés, avantageusement des acides aminés essentiels et des pigments, particulièrement des pigments photosynthétiques, dont les principaux sont les chlorophylles, et les caroténoïdes.

Il existe plusieurs sortes de chlorophylles, (chlorophylles a, b, c, d et f), qui diffèrent entre elles par les détails de leur structure moléculaire, et, d'autre part, par leurs propriétés spécifiques d'absorption. La chlorophylle "a" est le pigment photosynthétique le plus commun du règne végétal ; il se retrouve dans tous les végétaux terrestres et aquatiques. Ses pics d'absorption de la lumière se trouvent à 430 nm et à 660 nm. La chlorophylle "b" se retrouve chez les microalgues mais en quantité moindre. Ses pics d'absorption sont centrés à 450 nm et 645 nm.

Selon les paramètres d'illumination qui sont appliqués lors d'une culture de microalgue, il est possible d'orienter la production de métabolites et notamment de pigments. La combinaison d'une lumière discontinue sous forme de flashs et d'un spectre lumineux particulier peut être obtenue par l'emploi de LED (Light Emitting Diodes). Les longueurs d'ondes peuvent être choisies afin de correspondre ou se rapprocher du pic d'absorption des caroténoïdes ou des chlorophylles. L'induction de l'accumulation d'un pigment en particulier peut être obtenue tout en limitant l'accumulation des autres pigments présents dans la cellule.

Le terme de caroténoïde regroupe les carotènes (α, β, ε, γ, δ ou ζ-carotène, lycopène et phytoène) et les xanthophylles (astaxanthine, anthéraxanthine, citranaxanthine, cryptoxanthine, canthaxanthine, diadinoxanthine, diatoxanthine, flavoxanthine, fucoxanthine, lutéine, néoxanthine, rhodoxanthine, rubixanthine, siphonaxanthine, violaxanthine, zéaxanthine). Les caroténoïdes sont des pigments plutôt orange et jaune, liposolubles. Ils sont synthétisés par toutes les algues, toutes les plantes vertes et par de nombreux champignons et bactéries (dont les cyanobactéries). Ils sont absorbés par les animaux et les êtres humains dans leur nourriture. Les caroténoïdes ont deux principaux pics d'absorption situés autour de 440 et 475 nm.

Chez les plantes, ce sont des pigments accessoires de la photosynthèse, qui ont deux rôles principaux : un rôle de collecteur de lumière (transfert à la chlorophylle de l'énergie lumineuse qu'ils absorbent dans les gammes du spectre situées entre le violet et le rouge) et un rôle photoprotecteur (récupération de l'énergie de la chlorophylle en particulier en cas d'excès de lumière et d'ombre) dont la présence permet à la chlorophylle d'éviter les dégradations dues à la photooxydation.

Les caroténoïdes jouent un rôle important dans la nutrition et la santé, car plusieurs sont des provitamines A, et certains présentent aussi des activités anti-cancer et antioxydantes. Ils stimulent en outre la synthèse d'anticorps. Certains d'entre eux sont largement utilisés dans l'industrie agro-alimentaire pour leurs propriétés colorantes, et également dans les industries cosmétique et pharmaceutique pour leurs propriétés antioxydantes et leur capacité de photoprotection.

Certains caroténoïdes présentent un grand intérêt, comme la lutéine et l'astaxanthine, cette dernière particulièrement pour son important pouvoir antioxydant.

Les microalgues sont intéressantes comme source additionnelle de nourriture, car elles peuvent être source de protéines, de fibres, de lipides, ou encore d'agents antioxydants particulièrement des caroténoïdes. Elle peut être utilisée native, avantageusement séchée, mais aussi transformée, par exemple réduite sous forme de farines.

Elles peuvent être utilisées dans l'alimentation humaine ou animale, comme supplément nutritionnel ou incorporés en petite quantité dans des aliments, où elles peuvent remplacer les oeufs et les matières grasses, par exemple pour la panification.

Des farines de microalgues obtenues à partir d'algues dont la croissance a été réalisée dans des bassins ouverts ou dans des photobioréacteurs sont accessibles commercialement.

Il existe une souche de microalgues du genre Chlorelle (*chlorella protothecoides*) qui entre dans la composition de nombreux aliments, car cette souche présente la particularité de détruire sa chlorophylle en conditions d'hétérotrophie, c'est à dire quand le milieu contient une source de carbone. Il existe également des souches mutantes déficientes en pigments (US2013/0122181).

KR 2011 0072830 décrit un procédé autotrophe de culture de chlorelle qui utilise le CO₂ comme source de carbone, l'un des objectifs étant d'éliminer le CO₂ cause du réchauffement climatique. WO 2013/063075 indique qu'une diminution de l'intensité de l'éclairage entre 400 et 450 nm conduit à une stimulation de l'accumulation de chlorophylle.

Ainsi, hormis certains mutants et certaines souches sauvages d'algues telles que celles-ci-dessus décrites, la grande majorité des microalgues vertes présente un taux de chlorophylle important, ce qui les rend peu appréciées en raison de leur goût, et également du manque d'appétence de la couleur verte. Dès lors leur utilisation, comme précédemment décrit, est soit limitée, soit rendue complexe si l'on veut réduire la quantité de chlorophylle dans la biomasse utilisée dans l'utilisation désirée.

Il demeure donc un besoin en une biomasse de microalgues pauvre en chlorophylle mais riche en ses autres métabolites d'intérêt qui pourrait être issue de la culture de souches de microalgues commune et communément utilisée en culture.

Il serait donc intéressant de disposer d'un procédé de culture de microalgues "sauvages" (c'est-à-dire présentant normalement dans des conditions de culture standard un taux important de chlorophylle), procédé qui permettrait d'obtenir une biomasse desdites microalgues pauvre en chlorophylle mais riche en ses autres métabolites d'intérêt. Un tel procédé devrait permettre de réduire les quantités de pigments chlorophylliens lors de la culture des microalgues sauvages, présentant normalement en culture standard un taux important de chlorophylle.

La présente invention vise à fournir un tel procédé de culture de microalgues, qui pourrait permettre de mieux cibler la répartition des pigments dans les microalgues produites, afin d'obtenir des microalgues appauvries en chlorophylle, tout en maintenant naturellement un taux d'agent antioxydants, avantageusement de caroténoïdes élevé.

Une telle biomasse pourrait par la suite être utilisée comme il est connu, soit sous sa forme native, éventuellement séchée, soit sous la forme d'un dérivé comme par exemple sous la forme de farines, dans tous les domaines d'utilisation connus des microalgues ou de leurs dérivés (farines ou autres), particulièrement en nutrition, puis qu'appauvrie en chlorophylle, la dite biomasse ou ses dérivés présentant alors un goût et une couleur verte amoindris, voire inexistants, rendant ainsi les produits les incluant plus appétents.

Les inventeurs ont montré, de manière surprenante et après de longues recherches, qu'une culture de microalgues dans un milieu comprenant au moins une source de carbone, et comprenant au moins une étape d'éclairage par un rayonnement présentant un spectre étroit de longueur d'onde comprise entre 450 nm et 500 nm peut permettre d'obtenir une biomasse pauvre en chlorophylle et riche en agents antioxydants, particulièrement en caroténoïdes. En effet les inventeurs ont montré qu'un éclairage par un rayonnement présentant un spectre étroit centré à la longueur d'onde de 475 nm sera absorbé par les pigments caroténoïdes mais très peu par la chlorophylle.

Le procédé de culture réalisé avec un éclairage de ce type et avec un milieu de culture comprenant une source carbonée permet l'obtention d'une biomasse avec un contenu en chlorophylle inférieur à 500 ppm, voire moins de 100 ppm, préférentiellement 20 ou moins de 20 ppm, encore plus préférentiellement moins de 2 ppm, particulièrement chez certaines souches de microalgues, tout en maintenant une production de caroténoïdes normale voire améliorée, c'est-à-dire une quantité de caroténoïdes comprise entre 50 et 10000 ppm, 1000 et 7500 ppm, avantageusement entre 2000 et 5000 ppm..

Les microalgues, particulièrement certaines souches, produisent naturellement de la lutéine et en moindre quantité, de l'astaxanthine. Il est possible de favoriser la production d'astaxanthine par rapport à la lutéine en apportant une forte intensité lumineuse et/ou en utilisant un milieu carencé en azote (Cordero et al., Mar. Drugs 2012, 10, 2069-2088).

Avantageusement la production en astaxanthine peut être améliorée par rapport à la production de lutéine en apportant une forte intensité lumineuse et en utilisant un milieu carencé en azote.

Le procédé selon l'invention permet d'augmenter le contenu d'astaxanthine dans la biomasse obtenue de façon industrielle. De plus, la forte intensité lumineuse peut être limitée à la longueur d'onde choisie (475 nm) afin de limiter la production de chlorophylle.

Par étroit on entend selon l'invention que le spectre de lumière utilisé est centré sur une longueur d'onde donnée et ne s'étend de chaque côté de ladite longueur d'onde sur pas plus de 25 nm

Ainsi l'invention a pour objet premier un procédé de culture de microalgues dans un milieu de culture comprenant au moins une source de carbone, et comprenant au moins une étape d'éclairage par un rayonnement présentant un spectre étroit de longueur d'onde comprise entre 450 nm et 500 nm. De préférence selon l'invention, la longueur d'onde choisie sera comprise entre 460 et 490nm, très préférentiellement centrée à 475nm.

Un éclairage de la culture de microalgues par une lumière de longueur d'onde centrée à 475 nm et dont le spectre ne s'étend pas au-delà de 25 nm de chaque côté permet d'éviter l'accumulation de chlorophylle par les microalgues sans autre influence notable sur le développement des microalgues en culture, particulièrement sur l'accumulation des autres métabolites d'intérêt contenus dans lesdites microalgues, particulièrement des agents antioxydants, très particulièrement des caroténoïdes.

On comprend de ce qui précède que l'invention consiste en une variante de n'importe quel procédé de culture de microalgues connu, variante qui permet sur la base d'un procédé de culture connu d'obtenir une biomasse particulière. Cette variante (conditions d'éclairage particulières) peut donc être incluse dans n'importe quel procédé de culture de microalgues connu de l'art antérieur.

Ainsi selon l'invention, les conditions de culture des souches d'algues pourront être les conditions connues et utilisées pour cultiver les souches de microalgues utilisées. Avantageusement on utilisera les conditions qui permettront le meilleur rendement en biomasse. L'homme du métier saura intégrer l'étape d'éclairage selon l'invention dans un procédé connu, afin d'obtenir une biomasse la plus importante possible, qui réponde aux critères selon l'invention en taux de chlorophylle, et en taux de caroténoïdes.

A cet égard on pourra citer les procédés décrits par Cordero et al. (Marine Drugs, 2011, 9 :1607-1624) et Bumbak et al. (Appl Microbiol Biotechnol, 2011, 91 :31-46).

Il pourra être privilégié les procédés permettant un rendement en biomasse important. Comme exemple de procédé on pourra citer celui décrit par exemple par Doucha et Livansky (J Appl Phycol, 2012, 24 (1) : 35-43).

Plus particulièrement cette étape pourra être intégrée dans les procédés décrit par la demanderesse dans les demandes n°WO2013/136027 et WO2012/035262

Selon l'invention, l'éclairage peut être produit par tout moyen connu de l'homme du métier, notamment une ou plusieurs lampes, un ou plusieurs tubes, une ou plusieurs diodes électroluminescentes (DELs).

Les inventeurs ont démontré que le procédé est encore plus efficace lorsque l'éclairage est réalisé par une ou plusieurs diode(s) électroluminescente(s) (DEL).

Ainsi, selon une variante de l'invention, l'éclairage peut être réalisé par une ou plusieurs DELs. Les DELs sont de préférence des DELs du commerce.

A titre d'exemple on citera les DEL provenant de chez Seoul Optodevice Co., LTD (Corée du Sud), de chez Nichia Corporation (Japon), ou encore de chez SunLED Corporation (Etats-Unis).

Selon le procédé de l'invention ledit milieu de culture peut être soumis au rayonnement lumineux pendant un temps suffisant correspondant au moins au temps nécessaire pour que les critères de taux de chlorophylle et taux de caroténoïdes désirés soient remplis ; ce temps dépendra bien entendu du procédé de culture dans lequel cette étape d'éclairage sera incluse. L'homme du métier saura sans expérimentation excessive juger de ce temps nécessaire. Il saura adapter ce temps grâce à ses connaissances du domaine.

Plus particulièrement, les conditions de mixotrophie peuvent être obtenues dans des conditions d'éclairage discontinu et/ou variable au cours du temps.

Par éclairage discontinu, il faut entendre un éclairage ponctué par des périodes d'obscurité. L'éclairage peut être notamment sous forme de flashs. Un flash, au sens de l'invention, est un éclairage lumineux de durée donnée.

Selon l'invention, à propos de l'éclairage, 3 notions sont à considérer : la fréquence ou le nombre de flashs par unité de temps, la durée du flash et l'intensité de la lumière émise.

En termes de fréquence selon l'invention, on définit, selon le nombre de flashs par unité de temps utilisé dans le procédé selon l'invention deux types d'éclairage :
- Un éclairage à basse fréquence dont le nombre de flashs peut être compris entre environ 2 et 3,6 10⁴ par heure (5,4.10⁻⁴ Hz à 10 Hz), préférentiellement entre 3 et 3,6 10³ par heure (8,3.10⁻⁴ Hz à 1 Hz). On entend bien ici que le nombre de flashs par heure peut prendre toutes les valeurs comprises entre 2 et 36000 sans qu'il soit nécessaire de toutes les citer (2, 3, 4,..., 35598, 35599, 36000).
- Un éclairage à haute fréquence dont le nombre de flashs peut être compris entre environ 3,6 x10⁴ et 5,4 x 10⁹ (10 Hz à 1,5.10⁶ Hz) par heure, préférentiellement entre 3,6x10⁵ et 5,4x10⁹ (100 Hz à 1,5.10⁶ Hz). On entend bien ici que le nombre de flashs par heure peut prendre toutes les valeurs comprises entre 3,6 x10⁵ et 5,4 x 10⁹ sans qu'il soit nécessaire de toutes les citer (36000, 36001, 36002,..., 5399999998, 5399999999, 5400000000).

En termes de durée selon l'invention, quelle que soit la fréquence d'éclairage choisie, la durée du flash peut être comprise entre 1/150000 de seconde et 1799 secondes (29 minutes et 59 sec).

Bien évidement lorsque l'on utilise un éclairage à haute fréquence la durée du flash pourra être préférentiellement comprise entre 1/150000 de seconde et 1/10 de seconde. Et lorsque l'on utilise un éclairage à basse fréquence la durée du flash pourra être préférentiellement comprise entre 1/10 de seconde et 1799 secondes (29 minutes et 59 sec).

En termes d'intensité lumineuse selon l'invention, l'intensité de la lumière apportée sous forme de flashs peut être comprise entre 5 et 5000 µmol. m⁻². s⁻¹, de préférence entre 5 et 2000 µmol. m⁻². s⁻¹, ou 50 et 400 µmol. m⁻². s⁻¹, et plus préférentiellement entre 150 et 300 µmol. m⁻². s⁻¹ (1 µmol. m⁻². s⁻¹ correspond à 1µE m⁻². s⁻¹ (Einstein), unité souvent utilisée dans la littérature).

Selon l'invention le nombre de flashs par heure peut être choisi en fonction de l'intensité et de la durée des flashs (voir ci-dessus).

Selon l'invention les notions de fréquence, de durée et d'intensité lumineuse s'appliquent à l'éclairage tel que le prévoit l'invention, c'est-à-dire à l'éclairage produit par la source lumineuse choisie, avantageusement par une DEL, émettant un rayonnement lumineux de spectre étroit compris entre 450 et 500 nm, de préférence centré à 475 nm et pendant les temps considérés selon l'invention.

Selon un autre mode de l'invention, l'éclairage peut être variable, ce qui signifie que l'éclairage n'est pas interrompu par des phases d'obscurité, mais que l'intensité lumineuse varie au cours du temps. Cette variation de l'intensité de lumière est régulière et peut être périodique ou cyclique. Selon l'invention, on peut aussi procéder à un apport lumineux alliant des phases d'éclairage continues et discontinues.

Par éclairage variable, on entend que l'intensité de la lumière varie de manière régulière au moins deux fois par heure. Un exemple des conditions d'éclairage adaptées à la méthode d'invention est décrit dans la demande internationale de la Demanderesse publiée le 22 mars 2012 sous le N° WO2012035262.

L'éclairage peut présenter, de préférence, des variations d'intensité dont l'amplitude est généralement comprise entre 5 µmol. m⁻². s⁻¹ et 2000 µmol. m⁻². s⁻¹, de préférence entre 50 et 1500, plus préférentiellement entre 50 et 200 µmol. m⁻². s⁻¹.

Selon un mode de réalisation préféré, l'éclairage présente des variations d'intensité dont l'amplitude est comprise entre 5 et 1000 µmol.m⁻².s⁻¹, de préférence entre 5 et 400 µmol.m⁻².s⁻¹, ces variations ayant lieu entre 2 et 3600, de préférence entre 2 et 200 fois par heure.

Ces conditions de culture permettent d'apporter une quantité définie de lumière. Cet apport lumineux peut comporter des phases d'éclairage discontinu et/ou variable, avec des variations d'intensité pouvant avoir des amplitudes identiques ou différentes.

Avantageusement selon l'invention le procédé peut comprendre, simultanément ou indépendamment, toute autre étape nécessaire à la croissance de la biomasse comme par exemple sans être limitatif une ou plusieurs étape(s) de culture sans lumière ou encore une ou plusieurs étape(s) de récupération de la biomasse.

Le procédé selon l'invention découlera bien évidement du procédé choisi par l'homme du métier pour cultiver la souche d'algue choisie auquel il ajoutera une (ou plusieurs) étape(s) d'éclairage de la culture. Les conditions de culture seront donc dépendantes du procédé choisi. En particulier la température de culture dépendra de la souche choisie.

D'une manière générale la culture selon le procédé pourra s'effectuer à une température comprise entre 15°C et 38°C, avantageusement entre 22°C et 32°C.

Selon l'invention, les microalgues cultivées selon le procédé de l'invention peuvent être choisies parmi toutes les souches d'intérêt particulièrement parmi les classes des Chlorophyceae et des Trebouxiophyceae, qui sont deux classes d'algues vertes très proches phylogénétiquement (Lewis and McCourt, 2004 Am. J. Bot., 91 (10) : 1535-1556). La classification des microalgues, basée à l'origine sur des caractéristiques morphologiques et biochimiques, a été plusieurs fois remaniée pour intégrer les données génétiques au fur et à mesure de leur disponibilité. Il est apparu que certains genres comme Chlorella et Chlamydomonas sont polyphylétiques, c'est à dire qu'ils se retrouvent dans plusieurs classes. Par exemple, on retrouve des membres du genre chlorella dans les classes des Chlorophyceae et des Trebouxiophyceae. Parmi la classe des Chlorophyceae, les souches choisies appartiendront avantageusement aux genres Scenedesmus, Desmodesmus, Monoraphidium et Chlorella. Parmi la classe des Trebouxiophyceae, les souches choisis appartiendront avantageusement au genre Chlorella.

Lorsque les microalgues sont du genre Chlorella, elles pourront être choisies parmi les espèces *C. acuminata, C. anitrata, C. antarctica, C. botryoides, C. conductrix, C. conglomerata, C. desiccata, C. emersonii, C. fusca, C. glucotropha, C. homosphaera, C. infusionum, C. luteoviridis, C. marina, C. miniata, C. minutissima, C. mirabilis, C. nocturna, C. oocystoides, C. ovalis, C. parasitica, C. parva, C. peruviana, C. protothecoides, C. pyrenoidosa, C. regularis, C. rugosa, C. saccharophila, C. salina, C. sorokiniana, C. spaerckii, C. stigmatophora, C. subsphaerica, C. variabilis, C. variegata, C. vulgaris, C. xanthella, C. zopfingiensis.*

Avantageusement selon l'invention les algues du genre Chlorella pourront être des algues choisies parmi les espèces *C*. *sorokiniana, C. vulgaris* et *C*. *saccharophila.*

Lorsque les microalgues sont du genre Scenedesmus, elles pourront être choisies parmi les espèces *S*. *abundans, S. aciculatus, S. aculeolatus, S. aculeotatus, S. acuminatus, S. acutiformis, S. acutus, S. aldavei, S. ambuehlii, S. anhuiensis, S. anomalus, S. apicaudatus, S. apiculatus, S. arcuatus, S. aristatus, S. armatus, S. arvernensis, S. bacillaris, S. baculiformis, S. bajacalifornicus, S. balatonicus, S. basiliensis, S. bernardii, S. bicaudatus, S. bicellularis, S. bidentatus, S*. *bijuga, S. bijugatus, S. bijugus, S. brasiliensis, S. breviaculeatus, S. brevispina, S. caribeanus, S. carinatus, S. caudato-aculeolatus, S*. *caudatus, S*. *chlorelloides, S. circumfusus, S. coalitus, S. costatogranulatus, S. crassidentatus, S. curvatus, S. decorus, S. denticulatus, S. deserticola, S. dileticus, S. dimorphus, S. disciformis, S. dispar, S. distentus, S. ecornis, S. ellipsoideus, S. ellipticus, S. falcatus, S. fenestratus, S. grahneisii, S. granulatus, S. gujaratensis, S. hanleyi, S. helveticus, S. heteracanthus, S. hindakii, S. hirsutus, S. hortobagyi, S. huangshanensis, S. hystrix, S. incrassatulus, S. indianensis, S. indicus, S. intermedius, S*. *jovais, S. jugalis, S. kerguelensis, S. kissii, S. lefevrei, S. littoralis, S. tongispina, S. fongus, S. mirus, S. multistriatus, S. naegelii, S. nanus, S. oahuensis, S. obliquus, S. obtusus, S. olvalternus, S*. *opoliensis, S. ovalternus, S. pannonicis, S. papillosum, S. parisiensis, S*. *parvus, S*. *pecsensis, S. perforatus, S. planctonicus, S. plarydiscus, S. platydiscus, S. pleiomorphus, S. polessicus, S. polyglobulus, S. polyspinosus, S. praetervisus, S. prismaticus, S. producto-capitatus, S. protuberans, S. pseudoarmatus, S. pyrus, S. quadrialatus, S. quadricauda, S. quadrispina, S. raciborskii, S. reginae, S. reniformis, S*. *rostrato-spinosus, S. rotundus, S. schnepfii, S. securiformis, S. semipulcher, S. senilis, S. serrato-perforatus, S. serratus, S. serrulatus, S*. *setiferus, S. smithii, S*. *soli, S. sooi, S. spinosus, S. spinulatus, S. striatus., S. subspicatus, S. tetradesmiformis, S. tricostatus, S. tschudyi, S. vacuolatus, S. velitaris, S. verrucosus, S. vesiculosus, S. weberi, S. wisconsinensis, S. wuhanensis, S. wuhuensis.*

Avantageusement selon l'invention les algues du genre Scenedesmus pourront être des algues choisies parmi les espèces *S*. *abundans, S. armatus* et S. *obliquus.*

Lorsque les microalgues sont du genre Desmodesmus, elles pourront être choisies parmi les espèces *D. abundans (dans toutes ses variantes), D. aculeolatus, D. ambuehlii, D. armatus (dans toutes ses variantes), D. arthrodesmiformis, D. asymmetricus, D. baconii, D. bicaudatus, D. bicellularis, D. brasiliensis (dans toutes ses variantes), D. caudato-aculeatus (dans toutes ses variantes), D. communis (dans toutes ses variantes), D. costatogranulatus (dans toutes ses variantes), D. cuneatus, D. curvatocornis, D. denticulatus (dans toutes ses variantes), D. dispar, D. echinulatus, D. elegans, D. eupectinatus, D. fennicus, D. flavescens (dans toutes ses variantes), D. gracilis, D. grahneisii, D. granulatus, D. hystricoides, D. hystrix, D. insignis, D. intermedius (dans toutes ses variantes), D. itascaensis, D. kissii, D. komarekii (dans toutes ses variantes), D. lefevrei (dans toutes ses variantes), D. lunatus, D. magnus, D. maximus (dans toutes ses variantes), D. microspina, D. multicauda, D. multiformis, D. multivariabilis (dans toutes ses variantes), D. opoliensis (dans toutes ses variantes), D. pannonicus, D. perdix, D. perforates (dans toutes ses variantes), D. perforatus, D. pirkollei, D. pleiomorphus, D. polyspinosus, D. protuberans, D. pseudodenticulatus, D. pseudohystrix, D. pseudoserratus, D. quadricaudatus, D. regularis, D. santosii, D. schnepfii, D. serratoides, D. serrato-pectinatus, D. serratus, D. sp. CL1, D. sp. HegewalD. 1987*-*51*, *D. sp. ltas2*/*24S-1d, D. sp.* /*tas6*/*3T-2d, D. sp. Itas6*/*3T-2W, D. sp. Itas8*/*18S-6d, D. sp. Mary6*/*3T-2d, D. sp. NDem6*/*3P-3d, D. sp. NDem9*/*21T-10W, D. sp. Tow10*/*11T-12W, D. sp. Tow10*/*11T-17W, D. sp. Tow10*/*11T-1W, D. sp. Tow10*/*11T-2W, D. sp. Tow10*/*11T-3W, D. sp. Tow10*/*11T-6W, D. sp. Tow10*/*11T-8W, D. sp. Tow6*/*16T-10W, D. sp. Tow6*/*16T-15W, D. sp. Tow6*/*16T-16W, D. sp. Tow6*/*16T-17W, D. sp. Tow6*/*16T-26W, D. sp. Tow6*/*16T-31W, D. sp. Tow6*/*16T-32W., D. sp. Tow6*/*16T-35W, D. sp. Tow6*/*16T-8W, D. sp. Tow6*/*16T-9W, D. sp. Tow6*/*3T-11d, D. sp. Tow8*/*18P-13W, D. sp. Tow8*/*18P-14W, D. sp. Tow8*/*18P-1d, D. sp. Tow8*/*18P-25W, D. sp. Tow8*/*18P-3W, D. sp. Tow8*/*18P-4W, D. sp. Tow8*/*18T-10W, D. sp. Tow8*/*18T-23W, D. sp. Tow8*/*18T-25W, D. sp. Tow8*/*18T-5W, D. sp. WTwin8*/*18P-2d, D. spinosus, D. spinulatus, D. subspicatus (dans toutes ses variantes), D. tropicus (dans toutes ses variantes), D. ultrasquamatus.*

Avantageusement selon l'invention les algues du genre Desmodesmus pourront être des algues choisies parmi les espèces *D. istacaensis, D.intermedius et D. costado-granulatus.*

Lorsque les microalgues sont du genre *Monoraphidium,* elles pourront être choisies parmi les espèces *M*. *braunii, M*. *circinale, M. contortum, M. convolutum, M. dybowskii* (dans toutes ses variantes), *M. griffithii, M. minutum, M.neglectum, M.pusillum, M. saxatile, M. terrrestre, M. sp.* AKS-5, *M*. *sp.* Dek19, *M*. *sp.* FXY-10, *M.* sp. GK12, *M*. *sp.* IKA11, *M*. *Itas* (dans toutes ses variantes), *M.* sp. KMMCC (dans toutes ses variantes), *M. sp.* LUCC004, *M*. *sp*.N°5F4, *M. sp.* NTAI03, *M. sp.* PTP1, *M. sp.* PTP4, *M. sp.* SS.

Avantageusement selon l'invention les algues du genre Monoraphidium pourront être des algues choisies parmi les espèces *M*. *minutum, M. circinale* et *M*. *sp.* GK12.

Selon l'invention le procédé de culture peut être utilisé pour cultiver une seule souche de microalgues d'un genre donné, plusieurs souches d'un seul genre donné, ou plusieurs souches de genres différents donnés (au moins 2 espèces de 2 genres différents).

Selon l'invention la source carbonée peut être choisie parmi toute source carbonée connue et utilisable selon la souche choisie. L'homme du métier saura sans difficulté choisir la source carbonée la mieux adaptée à la souche à cultiver. A titre d'exemple on peut citer comme source carbonée utilisable le glucose, des dérivés de cellulose, du lactate, de l'amidon, du lactose, du saccharose, de l'acétate ou encore du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des cellules selon l'invention.

Les quantités de sources carbonées utilisées selon le procédé dépendront bien évidement de la souche choisie. L'homme du métier saura là encore sans difficulté adapter les quantités de source carbonée à la souche à cultiver sous forme pure ou en mélange.

Selon un mode de réalisation de l'invention, le substrat carboné organique pourra avoir une concentration comprise entre 5 mM et 1,5 M, de préférence 50 mM et 800 mM.

La culture peut être réalisée par toute technique de culture connue, par exemple en fioles ou en réacteur, mais aussi en fermenteurs ou encore dans tout contenant apte à la croissance de microalgues comme par exemple des bassins de type "raceway", à condition que ladite technique permette de mettre en contact les microalgues avec au moins la source carbonée, et en outre soit équipé d'au moins une source de lumière émettant dans les longueurs d'onde présentant un spectre étroit comprise entre 450 nm et 500 nm, de préférence comprise entre 460 et 490nm, très préférentiellement centrée à 475 nm, dont l'action sur la culture pourra conduire à la biomasse désirée c'est-à-dire une biomasse appauvrie en chlorophylle et riche en agents antioxydants, particulièrement riche en caroténoïdes (carotènes et xanthophylles, particulièrement lutéine et/ou astaxanthine).

Le procédé selon l'invention peut en outre comprendre une étape de récupération des microalgues. Ladite récupération des microalgues peut être réalisée par toute technique permettant la récupération de la biomasse, notamment les méthodes de filtration, gravimétrique ou sous pression réduite, de décantation, ou bien encore des méthodes de précipitation suivie d'une filtration gravimétrique.

Selon une variante de l'invention on pourra soumettre en outre la culture de microalgues à une carence en azote et/ou (simultanément ou indépendamment) à une forte intensité lumineuse.

Par carence en azote il est entendu selon l'invention une concentration en azote (sous toute forme connue) dans le milieu de culture qui pourra être comprise entre 0 et 0,5 mM, de préférence entre 0 et 100 µM.

Les inventeurs ont en effet montré que la culture de microalgues lorsque au moins la combinaison des facteurs suivants est réunie :
- présence d'une source de carbone ;
- soumission pendant un temps suffisant à au moins un éclairage présentant un spectre étroit de longueur d'onde comprise entre 450nm et 500nm ;
- concentration en azote réduite
- forte intensité lumineuse
conduit à la production d'une biomasse non seulement pauvre en chlorophylle et comprenant une quantité d'agents antioxydants, particulièrement des caroténoïdes, au moins normale voire améliorée, mais également dont la production en astaxanthine est favorisée par rapport à la production en lutéine.

L'astaxanthine a un pouvoir oxydant bien supérieur à la lutéine, stimule le système immunitaire, et présente des effets anti-inflammatoires. Il peut donc être donc très intéressant d'augmenter la quantité d'astaxanthine, même si cela suppose une baisse de la quantité de lutéine.

Plus précisément selon cette variante, la biomasse ainsi produite possède un taux de lutéine compris entre 10 et 1500 ppm, avantageusement entre 100 et 500 ppm et un taux d'astaxanthine compris entre 10 et 1000 ppm, avantageusement entre 200 et 500 ppm.

L'invention concerne également la biomasse susceptible d'être obtenue par l'une quelconque des variantes du procédé selon l'invention.

Les microalgues présentent un potentiel d'utilisation important dans beaucoup de domaines dont on citera par exemple la production de biocarburant, la dépollution, particulièrement des eaux usées, l'alimentation humaine ou animale, la cosmétique, la médecine.

La biomasse susceptible d'être obtenue selon le procédé de l'invention peut être bien évidement utilisée dans tous les domaines connus d'utilisation des microalgues, particulièrement en alimentation et/ou cosmétique.

On sait qu'une biomasse de microalgues susceptible d'être obtenue selon l'invention peut être utilisée après récolte soit directement, éventuellement séchée, soit après transformation. En particulier les biomasses de microalgues sont connues pour être utilisées sous la forme de farines entrant dans des compositions alimentaires ou sous forme de compléments alimentaires.

La biomasse de microalgues susceptible d'être obtenue selon l'invention peut être transformée en farine selon tout procédé connu de l'homme du métier. On peut ainsi envisager par exemple que les microalgues puissent être séparées du milieu de culture, lysées et réduites en particules fines (diamètre moyen de 10 microns), puis séchées.

L'invention concerne également toute utilisation de la biomasse de microalgues susceptible d'être obtenue selon l'invention dans tout domaine connu d'utilisation des microalgues, particulièrement la production de biocarburant, la dépollution, particulièrement des eaux usées, l'alimentation humaine ou animale, la cosmétique, la médecine, préférentiellement l'alimentation humaine ou animale, la cosmétique.

La biomasse obtenue après culture de microalgues selon le procédé de l'invention peut permettre d'obtenir en particulier une farine appauvrie en chlorophylle (moins de 500 ppm, avantageusement moins de 100 ppm, préférentiellement 20 ou moins de 20 ppm, très avantageusement moins de 2 ppm), riche en agents antioxydants, en particulier en caroténoïdes (carotènes et xanthophylles, particulièrement lutéine et/ou astaxanthine) en quantité comprise entre 10 et 1000 ppm, 1000 et 7500 ppm, avantageusement entre 2000 et 5000 ppm dont en particulier de la lutéine en une quantité comprise entre 1000 et 10000, avantageusement entre 2000 et 5000, et/ou de l'astaxanthine en une quantité comprise entre 10 et 1000 ppm, avantageusement entre 200 et 500 ppm, répondant à un besoin en particulièrement dans l'industrie alimentaire, parce que plus appétentes, ayant meilleur goût, apportant des antioxydants en quantité importante et pouvant être utilisable dans l'alimentation animale ou humaine.

Une farine susceptible d'être obtenue après transformation de la biomasse en microalgues susceptible d'être obtenue par le procédé selon l'invention est également divulguée.

Quelle que soit la forme d'utilisation du produit susceptible d'être obtenu par le procédé selon l'invention (biomasse native ou transformée), ledit produit peut être utilisé pur ou mélangé à d'autres ingrédients classiquement utilisés, particulièrement en alimentation ou en cosmétique.

L'invention concerne également tout produit pouvant comprendre au moins de la biomasse d'algues susceptible d'être obtenue selon l'invention.

Des produits pouvant comprendre au moins de la farine issue de la transformation de la biomasse d'algues susceptible d'être obtenue selon l'invention sont également divulgués.

Les exemples qui suivent illustrent la présente demande sans toutefois la limiter.

### Exemple 1 : Production de pigments chlorophyliens et caroténoides par les microalgues Scenedesmus et Desmodesmus.

Des cultures de *Scenedesmus abundans* et *Desmodesmus pannonicus* sont réalisées en flasques de 250 mL avec 50 mL de milieu de culture BG11 enrichi en azote (Rippka *et al*., 1979). Le substrat carboné utilisé est du glucose à 10 g/L. La température de culture est fixée à 26°C.

Les conditions d'éclairage sont les suivantes:
- Pour l'hétérotrophie : les cultures sont maintenues dans l'obscurité.
- Pour la mixotrophie: les cultures sont éclairées en flashs (0,5 hertz, 1800 flashs par heure) avec une intensité de 200 µmol.m⁻².s⁻¹. L'apport de lumière est obtenu par des lampes DEL dont la longueur d'onde s'étend de 450 nm à 500nm, le pic étant centré à 475 nm, pour la mixotrophie limitée à la lumière bleue.

Pour la mixotrophie « blanche », les DEL utilisées couvrent un spectre lumineux de 370 à 700 nm, avec 3 pics centrés à 445, 550 nm et 630 nm.

Après 7 jours de culture, les cellules sont centrifugées puis lyophilisées. Les méthodes d'extraction des pigments sont connues de l'homme du métier. Le tableau ci-dessous présente les résultats de 3 répliquats biologiques.

| Souche | Mode trophique | Chlorophylle a (ppm) | Chlorophylle b (ppm) | Lutéine (ppm) |
|---|---|---|---|---|
| *Scenedesmus abundans* | hétérotrophie | 99 (± 40) | 58 (± 29) | 68 (± 27) |
| *Scenedesmus abundans* | Mixotrophie "bleue" (450-500 nm) | 210 (± 31) | 304 (± 58) | 3189 (± 245) |
| *Scenedesmus abundans* | Mixotrophie "blanche" | 7705 (± 354) | 2101 (± 287) | 3568 (± 332) |
| *Desmodesmus pannonicus* | hétérotrophie | 270 (± 36) | 540 (±134) | 54 (± 11) |
| *Desmodesmus pannonicus* | Mixotrophie "bleue" (450-500 nm) | 235 (± 76) | 688 (± 66) | 1857 (±163) |
| *Desmodesmus pannonicus* | Mixotrophie "blanche" | 13886 (±204) | 4217 (±157) | 2289 (± 141) |

L'essai réalisé ici démontre les avantages du procédé selon l'invention. On constate en effet que la culture en Mixotrophie "bleue" permet d'obtenir une biomasse à faible teneur en chlorophylle a ou b par rapport à une culture en Mixotrophie "blanche" avec un taux de lutéine quasiment identique mais largement amélioré par rapport à une culture en hétérotrophie.

La Mixotrophie "bleue" combine les avantages de l'hétérotrophie en terme de quantité de chlorophylle a ou b et de la Mixotrophie blanche en termes de lutéine.

### Exemple 2 : Comparaison de l'effet de l'éclairage (longueur d'onde et flashs) sur la production de pigments par Chlorella protothecoïdes UTEX B25

### Composition du milieu :

| | | |
|---|---|---|
| Extrait de levure | 4 | g/L |
| MgSO₄, 7H₂0 | 500 | mg/L |
| KH₂PO₄ | 1 | g/L |
| CaCl₂ | 44 | mg/L |
| Solution stock Fe-EDTA (FeSO₄ à 6,9 g/L et d'EDTA-Na₂ à 9,3 g/L) | 3 | mL/L |
| Solution de trace métal (3,09 g/L EDTA-Na₂ ; 0,080 g/L CuSO₄, 5H₂O ; 2,860 g/L H₃BO₃ ; 0,040 g/L NaVO₃, 4H₂O ; 1,820 g/L MnCl₂ ; 0,040 g/L CoCl₂,6H₂O ; 0,220 g/L ; ZnSO₄,7H₂O ; 0,017 g/L Na₂SeO₃ ; 0,030 g/L (NH₄)₆Mo₇O₂₄, 4H₂O) | 4 | ml/L |
| Glucose | 30 | g/L |

### Conditions de culture :

Dans chaque Erlenmeyer on inocule 100 ml de milieu à 1% avec une pré-culture de *Chlorella protothecoïdes* âgée de 7 jours.

Pour tester l'effet de la lumière on illumine de façon indépendante les erlenmeyers avec un système de LED blanche ou de LED bleue à 455 nm ou à 475 nm. L'intensité lumineuse pour chacune des conditions est de 0 µmol m⁻² s⁻¹ (µE) en condition hétérotrophe, 100 µmol m⁻² s⁻¹ avec un fréquence de 1 seconde d'éclairement toutes les 10 secondes, et 5000 µmol m⁻² s⁻¹ avec un fréquence de 1 seconde d'éclairement toutes les 60 secondes en conditions de mixotrophie. Les cellules sont cultivées à une température de 26°C sous agitation modérée (200 rpm). Le suivi de la croissance des cellules est effectué toutes les 24h par mesure d'absorbance à 800 nm. Lorsque la phase stationnaire est atteinte (7 jours) 50 mL de suspension cellulaire sont prélevés afin d'effectuer l'analyse des quantités de pigments, chlorophylle et caroténoïdes contenus dans la biomasse.

| | Caroténoïdes (ppm) | | | |
|---|---|---|---|---|
| Intensité lumineuse et flash | Lumière blanche | Lumière bleue (455 nm) | Lumière bleue (475 nm) | Hétérotrophie |
| 0 µE | - | - | - | 58 |
| 100 µE/1 sec toutes les 10 s | 120 | 109 | 101 | - |
| 5000 µE/1 sec toutes les 60 s | 180 | 134 | 162 | - |

| | Chlorophylles (ppm) | | | |
|---|---|---|---|---|
| Intensité lumineuse et flash | Lumière blanche | Lumière bleue (455 nm) | Lumière bleue (475 nm) | Hétérotrophie |
| 0 µE | - | - | - | 0 |
| 100 µE/1 sec toutes les 10 s | 81 | 0 | 0 | - |
| 5000 µE/1 sec toutes les 60 s | 200 | 106 | 20 | - |

Les résultats montrent qu'en absence de lumière (0 µE) il y a absence de chlorophylle et présence de caroténoïdes. En présence de flash de faible intensité (100 µE) la quantité de caroténoïdes dans la biomasse est plus élevée qu'en hétérotrophie quelle que soit les longueurs d'ondes utilisées. Toutefois, on peut noter qu'en lumière bleue (455 nm ou 475 nm) il n'y a pas production de chlorophylle contrairement à ce qui est observé en lumière blanche. Les flashs de très forte intensité et de basse fréquence semblent avoir un effet accru sur la production de caroténoïdes par rapport au flash de faible intensité et de plus haute fréquence. Il faut noter qu'en présence de lumière blanche et de lumière bleue à 455 nm la quantité de chlorophylle augmente de façon importante 200 et 106 ppm respectivement alors qu'à 475 nm la quantité de chlorophylle est 10 à 5 fois moins importante.

## Revendications

1. Procédé de culture de microalgues choisies parmi les genres Chlorella, Scenedesmus, Desmodesmus et Monoraphidium dans un milieu de culture comprenant au moins une source de carbone choisie parmi le glucose, des dérivés de cellulose, du lactate, de l'amidon, du lactose, du saccharose, de l'acétate ou du glycérol pour la préparation d'une biomasse ayant un contenu en chlorophylle inférieure à 500 ppm et une quantité de caroténoïdes comprise entre 50 et 10000 ppm, **caractérisé en ce qu'**il comprend au moins une étape d'éclairage par un rayonnement présentant un spectre étroit de longueur d'onde comprise entre 450 nm et 500 nm et centrée à 475 nm.

2. Procédé selon la revendication 1, caractérisé en ce le rayonnement présente un spectre étroit de longueur d'onde comprise entre 460 nm et 490 nm.

3. Procédé selon l'une quelconque des revendications 1 ou 2, **caractérisé en ce que** l'éclairage est apporté sous forme de flashes.

4. Procédé selon l'une quelconque des revendications 1 ou 2, **caractérisé en ce que** l'éclairage n'est pas interrompu par des phases d'obscurité et que l'intensité lumineuse varie au cours du temps.

5. Procédé selon l'une quelconque des revendications 1 à 4, **caractérisé en ce que** la culture est réalisée à une température comprise entre 15°C et 38°C, avantageusement entre 22°C et 32°C

6. Procédé selon l'une quelconque des revendications 1 à 5, **caractérisé en ce qu'**en outre la culture de microalgues est soumise à une carence en azote, la concentration en azote dans le milieu de culture étant comprise entre 0 et 0,5 mM.

7. Biomasse de microalgues choisies parmi les genres Chlorella, Scenedesmus, Desmodesmus et Monoraphidium ayant un contenu en chlorophylle inférieur à 500 ppm et une quantité de caroténoïdes comprise entre 50 et 10000 ppm.

8. Biomasse selon la revendication 7, **caractérisée en ce qu'**elle présente un taux de lutéine compris entre 1000 et 10000 ppm.

9. Biomasse selon l'une quelconque des revendications 7 ou 8, **caractérisée en ce qu'**elle présente un taux d'astaxanthine compris entre 10 et 1000 ppm.

10. Utilisation de la biomasse telle que décrite à l'une quelconque des revendications 7 à 9, pour la production de biocarburant, la dépollution, particulièrement des eaux usées, l'alimentation humaine ou animale, la cosmétique, la médecine, préférentiellement l'alimentation humaine ou animale, la cosmétique.

11. Utilisation de la biomasse telle que décrite à l'une quelconque des revendications 7 à 9, pour la production d'une farine.

12. Produit comprenant au moins de la biomasse telle que décrite dans l'une quelconque des revendications 7 à 9.

13. Procédé de préparation d'une farine comprenant la transformation de la biomasse de microalgues telle que décrite dans l'une quelconque des revendications 7 à 9 par séparation des microalgues du milieu de culture, lyse et réduction en particules fines puis séchage.

## Patentansprüche

1. Verfahren zur Kultivierung von Mikroalgen, ausgewählt aus den Gattungen Chlorella, Scenedesmus, Desmodesmus und Monoraphidium in einem Kulturmilieu, umfassend mindestens eine Carbonquelle, ausgewählt aus der Glucose, Zellulosederivaten, Laktat, Stärke, Laktose, der Saccharose, Acetat oder Glycerol für die Herstellung einer Biomasse mit einem Inhalt an Chlorophyll unter 500 ppm und einer Carotinoidmenge, die zwischen 50 und 10000 ppm liegt, **dadurch gekennzeichnet, dass** es mindestens einen Beleuchtungsschritt durch eine Strahlung umfasst, die ein enges Spektrum mit einer Wellenlänge aufweist, die zwischen 450 nm und 500 nm liegt und auf 475 nm zentriert ist.

2. Verfahren nach Anspruch 1, **dadurch gekennzeichnet, dass** die Strahlung ein enges Spektrum mit einer Wellenlänge aufweist, die zwischen 460 nm und 490 nm liegt.

3. Verfahren nach einem der Ansprüche 1 oder 2, **dadurch gekennzeichnet, dass** die Beleuchtung in Form von Blitzen zugeführt wird.

4. Verfahren nach einem der Ansprüche 1 oder 2, **dadurch gekennzeichnet, dass** die Beleuchtung nicht durch Dunkelphasen unterbrochen wird und dass die Lichtintensität im Laufe der Zeit schwankt.

5. Verfahren nach einem der Ansprüche 1 bis 4, **dadurch gekennzeichnet, dass** die Kultur bei einer Temperatur hergestellt wird, die zwischen 15 °C und 38 °C, in vorteilhafter Weise zwischen 22 °C und 32 °C, liegt.

6. Verfahren nach einem der Ansprüche 1 bis 5, **dadurch gekennzeichnet, dass** die Mikroalgenkultur einer Stickstoffkarenz unterzogen wird, wobei die Stickstoffkonzentration im Kulturmilieu zwischen 0 et 0,5 mM liegt.

7. Biomasse aus Mikroalgen, ausgewählt aus den Gattungen Chlorella, Scenedesmus, Desmodesmus und Monoraphidium mit einem Chlorophyllgehalt unter 500 ppm und einer Carotinoidmenge zwischen 50 und 10000 ppm.

8. Biomasse nach Anspruch 7, **dadurch gekennzeichnet, dass** sie einen Luteingehalt aufweist, der zwischen 1000 und 10000 ppm liegt.

9. Biomasse nach einem der Ansprüche 7 oder 8, **dadurch gekennzeichnet, dass** sie einen Astaxanthingehalt aufweist, der zwischen 10 und 1000 ppm liegt.

10. Verwendung der Biomasse wie nach einem der Ansprüche 7 bis 9 beschrieben für die Herstellung von Biokraftstoff, die Reinigung, insbesondere von Abwässern, die Ernährung von Mensch oder Tier, die Kosmetik, die Medizin, vorzugsweise die Ernährung von Mensch oder Tier, die Kosmetik.

11. Verwendung der Biomasse wie nach einem der Ansprüche 7 bis 9 beschrieben für die Herstellung eines Mehls.

12. Produkt, umfassend mindestens Biomasse wie nach einem der Ansprüche 7 bis 9 beschrieben.

13. Verfahren zur Herstellung eines Mehls, umfassend die Umwandlung der Mikroalgen-Biomasse wie nach einem der Ansprüche 7 bis 9 beschrieben durch Trennen der Mikroalgen von dem Kulturmilieu, Lyse und Reduzieren in feine Partikel, dann Trocknen.

## Claims

1. Process for culturing microalgae selected from the genera *Chlorella, Scenedesmus, Desmodesmus* and *Monoraphidium* in a culture medium comprising at least one carbon source selected from glucose, cellulose derivatives, lactate, starch, lactose, sucrose, acetate or glycerol for the preparation of biomass having a chlorophyll content of less than 500 ppm and a quantity of carotenoids comprised between 50 and 10000 ppm, **characterized in that** it comprises at least one step of illumination with radiation having a narrow wavelength spectrum comprised between 450 nm and 500 nm and centred at 475 nm.

2. Process according to claim 1, **characterized in that** the radiation has a narrow wavelength spectrum comprised between 460 nm and 490 nm.

3. Process according to any one of claims 1 or 2, **characterized in that** the illumination is provided in the form of flashes.

4. Process according to any one of claims 1 or 2, **characterized in that** the illumination is not interrupted by phases of darkness and that the light intensity varies over time.

5. Process according to any one of claims 1 to 4, **characterized in that** the culture is carried out at a temperature comprised between 15°C and 38°C, advantageously between 22°C and 32°C

6. Process according to any one of claims 1 to 5, **characterized in that** in addition the microalgae culture is subjected to nitrogen deficiency, the nitrogen concentration in the culture medium being between 0 and 0.5 mM.

7. Biomass of microalgae selected from the genera *Chlorella, Scenedesmus, Desmodesmus* and *Monoraphidium* having a chlorophyll content of less than 500 ppm and a quantity of carotenoids comprised between 50 and 10000 ppm.

8. Biomass according to claim 7, **characterized in that** it has a lutein level comprised between 1000 and 10000 ppm.

9. Biomass according to any one of claims 7 or 8, **characterized in that** it has an astaxanthin level comprised between 10 and 1000 ppm.

10. Use of the biomass as described in any one of claims 7 to 9, for biofuel production, environmental remediation, particularly of wastewater, human food or animal feed, cosmetics, medicine, preferentially human food or animal feed, cosmetics.

11. Use of the biomass as described in any one of claims 7 to 9, to produce flour.

12. Product comprising at least the biomass as described in any one of claims 7 to 9.

13. Process for preparing a flour comprising transforming the microalgae biomass as described in any one of claims 7 to 9 by separating the microalgae from the culture medium, lysing and reducing to fine particles and then drying.
